# EUROPÄISCHE PATENTANMELDUNG

(11) **EP 1 267 296 A2**
(43) Veröffentlichungstag der Anmeldung: **18.12.2002**
(21) Anmeldenummer: 02012116.6
(22) Anmeldetag: 31.05.2002
(51) Int. Cl.: G06F 19/00, G06F 17/30, G06F 17/60

(54) **Verfahren und Datenbank zum Ermitteln einer einen medizinischen Befund erstellenden Institution**

(30) Priorität: 13.06.2001 DE 10128524
(71) Anmelder: SIEMENS AKTIENGESELLSCHAFT, 80333 München (DE)
(72) Erfinder: Christ, Tilo, 91058 Erlangen (DE); Prihoda, Heinz, 90562 Heroldsberg (DE); Schmidt, Volker, Dr., 91054 Erlangen (DE); Schneider, Siegfried, Dr., 91056 Erlangen (DE); Schüll, Hans-Dieter, 91085 Weisendorf (DE); Striebel, Werner, 90592 Schwarzenbruck (DE); Zahlmann, Gudrun, Dr., 92318 Neumarkt (DE)

(57) **Zusammenfassung**

Die Erfindung betrifft ein Verfahren zum Ermitteln einer einen medizinischen Befund erstellenden Institution, aufgrund dessen eine Datenbank (10) über ein Informationsübertragungsnetz einen ersten Auftrag zum Ermitteln einer Institution (20, 30) empfängt. Der erste Auftrag umfasst einen ersten medizinischen Datensatz eines ersten Patienten (54, 64, 74a-74y), aufgrund dessen ein erster medizinischer Befund erstellt werden soll. In der Datenbank (10) sind Informationen über medizinische Befunde erstellende Institutionen (20, 30) gespeichert, die aufgrund des ersten Auftrages abgefragt werden. Basierend auf den abgefragten Informationen wird eine geeignete Institution (20, 30) zum Erstellen des ersten medizinischen Befundes ermittelt und der erste Auftrag mit dem ersten Datensatz an diese Institution (20, 30) weiter geleitet. Die Erfindung betrifft außerdem eine geeignet konfigurierte Datenbank, mit der das Verfahren durchführbar ist.

## Beschreibung

Die Erfindung betrifft ein Verfahren zum Ermitteln einer einen medizinischen Befund erstellenden Institution und eine Datenbank.

Bisweilen ist ein einen Patienten untersuchender Arzt nicht sicher, ob eine von ihm gestellte Diagnose für den Patienten richtig ist. Dann kann er einen medizinischen Datensatz, den er während der Untersuchung des Patienten ermittelte, an einen weiteren Arzt schicken, so dass dieser für ihn einen medizinischen Befund des Patienten erstellen kann. Ein Beispiel einer solchen Vorgehensweise ist, wenn ein Allgemeinmediziner ein Röntgenbild der Lunge des Patienten herstellt und sich unschlüssig über die Diagnose ist, da er kein Lungenexperte ist. Dann kann er z.B. das Röntgenbild einem Lungenexperten übermitteln, so dass dieser den medizinischen Befund erstellt.

Es ist jedoch nicht immer möglich, insbesondere schnell und in einfacher Weise einen geeigneten Experten zu finden, der den medizinischen Befund erstellen kann.

In der WO 98/41943 ist ein medizinisches Management System beschrieben, mit dessen Hilfe ein Allgemeinmediziner mit einem Client-Rechner oder einem Terminal eine Anfrage für eine medizinische Beratung an einen Host Rechner schicken kann. Die Anfrage umfasst Patientendaten, wie z.B. medizinische Bilder, Labortests, usw.. Die empfangene Anfrage wird von einem Arzt gelesen, der einen geeigneten Spezialisten auswählt und die Anfrage an den Spezialisten weiterleitet. Auf die Anfrage empfängt der Host-Rechner einen Kommentar des Spezialisten, der an den Allgemeinmediziner weitergeleitet wird.

Der WO 00/70529 ist ein interaktives, Netzwerk basiertes Informationssystem, das aktuelle medizinische Informationen zur Verfügung stellt, offenbart. Die bereitgestellte Information ist einem Benutzer des Informationssystems angepasst. Das Informationssystem ist für eine Fernüberwachung oder eine Ferndiagnose eines Patienten geeignet.

Die Aufgabe der Erfindung ist daher, eine Voraussetzung zu schaffen, dass eine geeignete Institution in einfacher Weise ermittelt wird, die einen medizinischen Befund aufgrund vorhandener medizinischer Daten erstellt.

Die Aufgabe der Erfindung wird gelöst durch ein Verfahren zum Ermitteln einer einen medizinischen Befund erstellenden Institution, aufweisend folgende Verfahrensschritte:
- Empfangen eines ersten Auftrages mit einer Datenbank, wobei der erste Auftrag über ein Informationsübertragungsnetz an die Datenbank übermittelt wurde, der erste Auftrag einen ersten medizinischen Datensatz eines ersten Patienten umfasst und der erste Auftrag ein Auftrag zum Erstellen eines ersten medizinischen Befundes aufgrund des ersten medizinischen Datensatzes ist,
- basierend auf dem ersten Auftrag, automatisches Abfragen von in der Datenbank gespeicherten Informationen über medizinische Befunde erstellende Institutionen aus einer Menge von Institutionen,
- basierend auf den abgefragten Informationen, Ermitteln einer geeigneten Institution aus der Menge der Institutionen, die den ersten medizinischen Befund aufgrund des ersten Datensatzes erstellen soll,
- Weiterleiten des ersten Auftrages mit dem ersten Datensatz an die geeignete Institution.

An einem ersten Ort ist der erste medizinische Datensatz des ersten Patienten verfügbar, der dazugehörige erste medizinische Befund soll aber an einem anderen Ort erstellt werden. Daher soll die geeignete Institution gefunden werden, die aufgrund des ersten medizinischen Datensatzes den ersten medizinischen Befund erstellt. Dies ist z.B. nötig, wenn, wie bereits in der Einleitung beschrieben, ein Arzt über eine von ihm erstellte Diagnose unschlüssig ist und eine zweite Meinung insbesondere von einem Experten erhalten möchte. Erfindungsgemäß wird der erste Auftrag von dem ersten Ort, der z.B. die Arztpraxis des eben genannten Arztes ist, an die Datenbank übermittelt. Die Datenbank ermittelt die geeignete Institution, die aufgrund des ersten Auftrages den ersten medizinischen Befund erstellen kann. Die Ermittlung der geeigneten Institution erfolgt in automatisierter Weise. Folglich muss sich der eben genannte Arzt nur an eine Stelle wenden, wenn er einen medizinischen Befund erstellt haben möchte, was insbesondere dann vorteilhaft ist, wenn er mehrere medizinische Befunde erstellt haben möchte. Das Informationsübertragungsnetz, mit dem der erste Auftrag an die Datenbank übermittelt wird, ist beispielsweise das Internet.

Nach einer Ausführungsform der Erfindung wird der erste Auftrag nach der Übermittlung an die Datenbank in eine Liste eingetragen, wobei die Liste Aufträge umfasst, für die jeweils noch keine geeignete Institution ermittelt wurde, und wobei die Liste von Institutionen der Menge von Institutionen eingesehen werden kann, um ein Angebot zum Erstellen des medizinischen Befundes an den zentralen Ort zu übermitteln. Somit haben die Institutionen die Möglichkeit, sich über Aufträge zu informieren, die an die Datenbank übermittelt wurden, aber noch nicht an eine Institution weitergegeben worden sind. Daraufhin können diese Institutionen an die Datenbank ein Angebot zum Erstellen des medizinischen Befundes übermitteln.

Die Liste kann gemäß einer vorteilhaften Variante der Erfindung über das Internet aufgerufen werden.

Nachdem die ermittelte Institution den ersten medizinischen Befund erstellt hat, kann sie gemäß Ausführungsformen der Erfindung den ersten medizinischen Befund direkt an den Auftraggeber des ersten medizinischen Befundes oder an die Datenbank übermitteln, so dass der medizinische Befund von der Datenbank an den Auftraggeber weitergeleitet wird. Es ist insbesondere dann vorteilhaft für die Institution, den ersten medizinischen Befund an die Datenbank zu übermitteln, wenn sie mehrere medizinische Befunde für verschiedene Auftraggeber erstellt hat. Dann muss sie nämlich nicht mehrere medizinische Befunde an verschiedene Orte, sondern kann die medizinischen Befunde gebündelt an den zentralen Ort der Datenbank übermitteln. Ferner kann auch die Identität der Institution dem Auftraggeber geheim gehalten werden.

Nach einer Ausführungsform der Erfindung ist die Institution ein Arzt, ein Ärzteteam und/oder ein Krankenhaus. So kann sich beispielsweise ein Arzt oder ein Ärzteteam auf die Erstellung medizinischer Befunde für weitere Personen spezialisieren.

Gemäß einer besonders bevorzugten Variante der Erfindung umfasst der ersten medizinischen Datensatz ein Bild, einen Videofilm, Laborergebnisse, ein EKG und/oder eine Krankenakte des ersten Patienten. Das Bild wird dabei gemäß einer Ausführungsform der Erfindung von einem bildgebenden medizintechnischen Gerät hergestellt, welches gemäß einer besonders bevorzugten Ausführungsform der Erfindung ein Computertomograph, ein Magnetresonanzgerät, ein Ultraschallgerät oder ein Röntgengerät ist.

Gemäß einer besonders bevorzugten Ausführungsform der Erfindung wird die geeignete Institution basierend auf wenigstens einem Kriterium ermittelt.

Das Kriterium kann gemäß einer Variante der Erfindung eine geforderte Qualität des erstellten ersten medizinischen Befundes, eine medizinische Spezialisierung der Institution, eine Zeitdauer zur Erstellung des ersten medizinischen Befundes, eine Anzahl von medizinischen Befunden, die die Institution in einer vorgegebenen Zeitdauer erstellen kann, von der Institution für die Erstellung des ersten medizinischen Befundes geforderte Gebühr und/oder gesetzliche Regelungen sein. Deshalb kann z.B. garantiert werden, dass die ermittelte Institution einen qualitativ hochwertigen ersten medizinischen Befund erstellt oder nötigenfalls den ersten medizinischen Befund schnell erstellt und dass eine kostengünstige Institution zur Erstellung des ersten medizinischen Befundes ermittelt wird.

Das Kriterium kann aber auch nach einer Ausführungsform der Erfindung eine Qualität des von der Datenbank empfangenen ersten medizinischen Datensatzes sein.

Wenn nach einer Ausführungsform der Erfindung die Datenbank eine von der ermittelten Institution erbrachten Leistung überwacht, ist eine Vorraussetzung geschaffen, dass die ermittelte Institution gleichbleibende medizinische Befunde hoher Qualität erstellt.

Die erbrachte Leistung kann gemäß einer Variante der Erfindung eine Qualität des ersten erstellten medizinischen Befundes, ein Einhalten einer vorgegebenen Zeitdauer für die Erstellung des ersten medizinischen Befundes und/oder eine Einhaltung einer Erstellung einer vorgegebenen Anzahl von medizinischen Befunden sein.

Das erfindungsgemäße Verfahren kann dann besonders praktisch für den Auftraggeber oder für die Institution, die den ersten medizinischen Befund erstellt, ausgeführt werden, wenn eine Rechnung für den erstellten ersten medizinischen Befund von der Datenbank erstellt und an den Auftraggeber übermittelt wird. Dies ist dann besonders praktisch, wenn die Institution viele medizinische Befunde erstellt oder von dem ersten Ort viele medizinische Datensätze von verschiedenen Patienten übermittelt werden.

Gemäß einer bevorzugten Variante der Erfindung ist vorgesehen, dass der erste Auftrag ein Auftrag zum Erstellen mehrerer medizinischer Befunde für mehrere Patienten ist, der für die Erstellung der medizinischen Befunde notwendige medizinische Datensätze umfasst und die medizinischen Befunde von einer Institution oder von mehreren Institutionen aus der Menge von Institutionen erstellt werden. Gerade wenn eine Erstellung vieler medizinischer Befunde von einer Person in Auftrag gegeben wird, kann es möglich sein, dass eine Institution nicht alle Befunde insbesondere in einer geforderten Zeit erstellen kann. Dann ist es besonders vorteilhaft, wenn der erste Auftrag von der Datenbank in mehrere kleinere Aufträge aufgeteilt und an mehrere Institutionen weiter geleitet wird.

Nach einer weiteren bevorzugten Ausführungsform der Erfindung empfängt zusätzlich die Datenbank einen zweiten Auftrag zum Erstellen eines zweiten medizinischen Befundes eines zweiten Patienten über das Informationsübertragungsnetz. Der zweite Auftrag umfasst einen für die Erstellung des zweiten medizinischen Befundes notwendigen zweiten medizinischen Datensatz des zweiten Patienten. Die Datenbank fasst den erste und den zweiten Auftrag zu einem Sammelauftrag zum Erstellen des ersten und des zweiten medizinischen Befundes zusammen und leitet den Sammelauftrag an eine Institution aus der Menge von Institutionen weiter. Dies ist insbesondere dann vorteilhaft, wenn eine Institution einen Preisnachlass gewährt, wenn sie einen Auftrag zum Erstellen mehrerer medizinischer Befunde erhält.

Die Aufgabe der Erfindung wird auch gelöst durch eine Datenbank,
- die über ein Informationsübertragungsnetz kontaktierbar ist,
- an die ein Auftrag zum Erstellen eines medizinischen Befundes aufgrund eines den Auftrag umfassenden medizinischen Datensatzes eines Patienten über das Informationsübertragungsnetz übermittelbar ist,
- in der Daten einer Menge von Institutionen, die medizinische Befunde erstellen, gespeichert und abfragbar sind,
- die derart konfiguriert ist, dass sie automatisch die Daten der Menge von Institutionen mit dem Auftrag vergleicht und eine zum Erstellen des ersten medizinischen Befundes geeignete Institution aus der Menge von Institutionen auswählt, und
- und die derart konfiguriert ist, dass sie den medizinischen Datensatz über das Informationsübertragungsnetz an die ermittelte Institution weiterleitet.

Das erfindungsgemäße System ist also derart ausgeführt, dass mit ihm auch das erfindungsgemäße Verfahren durchgeführt werden kann.

Vorteilhafte Ausgestaltungen der Erfindung ergeben sich aus den Unteransprüchen.

Ein Ausführungsbeispiel ist exemplarisch in den schematischen Zeichnungen dargestellt. Es zeigen:
- Fig. 1: ein zur Veranschaulichung des erfindungsgemäßen Verfahrens geeignetes Szenarium,
- Fig. 2: ein erfindungsgemäßes System und

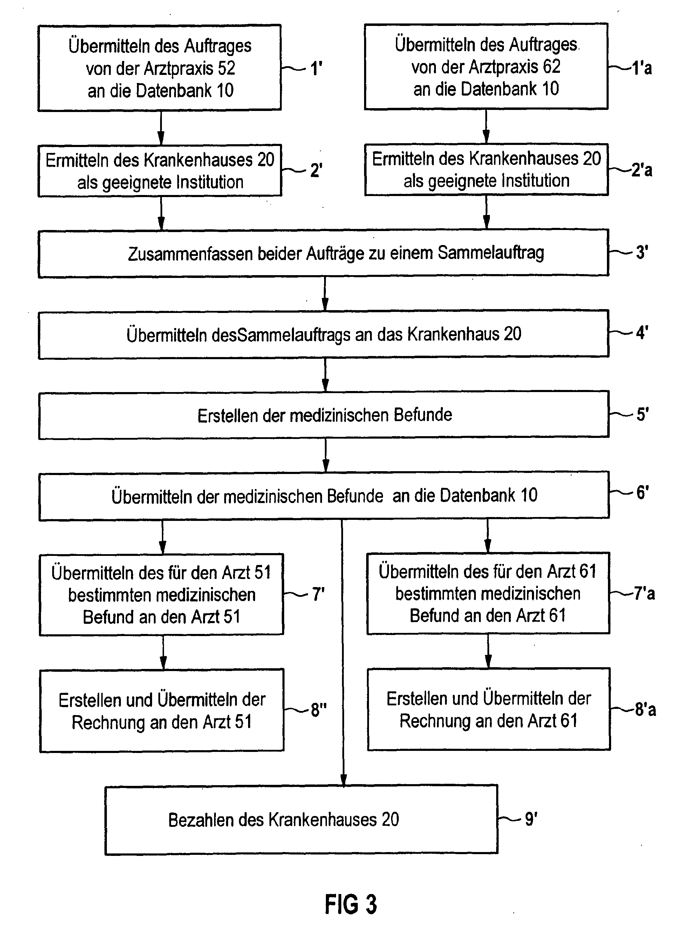

- Fig. 3 und 4: Flussdiagramme zur Veranschaulichung des erfindungsgemäßen Verfahrens.

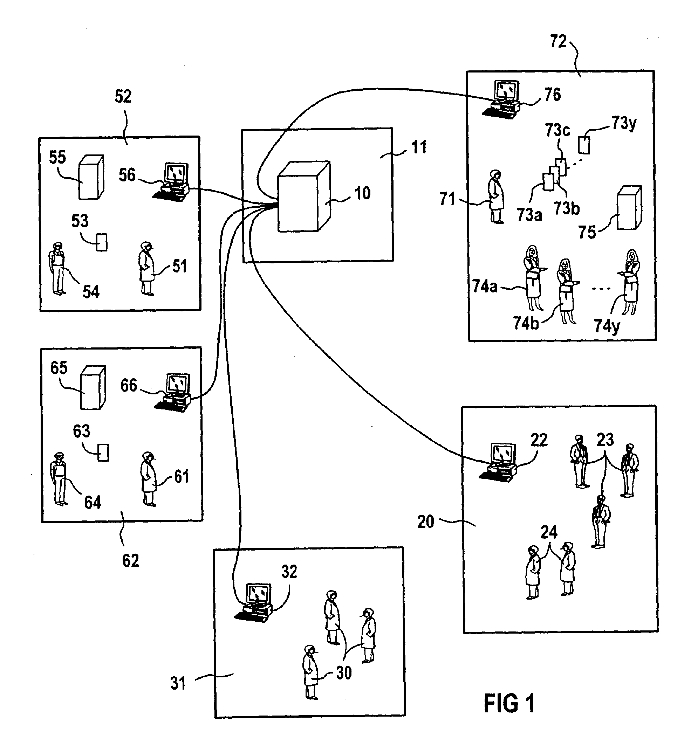

Die Fig. 1 zeigt eine im Falle des vorliegenden Ausführungsbeispiels an das Internet angeschlossene Datenbank 10, die sich in einem Geschäftsraum 11 eines telemedizinischen Dienstleisters befindet und auch von dem telemedizinischen Dienstleister betrieben wird.

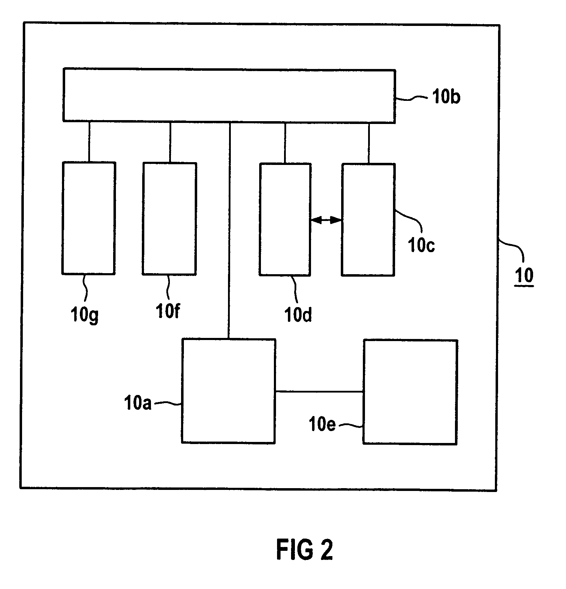

In der Datenbank 10 ist ein geeignetes Rechnerprogramm gespeichert, dass aufgrund von in der Datenbank 10 gespeicherter Daten und eines an die Datenbank 10 übermittelten Auftrages eine geeignete Institution ermittelt, die einen aufgrund des Auftrages geforderten medizinischen Befund erstellen kann. Ein möglicher Aufbau der Datenbank 10 bzw. des in der Datenbank 10 gespeicherten Rechnerprogramms ist exemplarisch und schematisch in der Fig. 2 dargestellt.

Wenn ein Auftrag an die Datenbank 10 übermittelt wird, wird der Auftrag zunächst in einem Speichermodul 10a gespeichert. Anschließend vergleicht ein Vermittlungsmodul 10b den übermittelten und in dem Speichermodul 10a gespeicherten Auftrag mit einem in der Datenbank 10 gespeicherten Leistungskatalog 10c. Der Leistungskatalog 10c umfasst Angaben über Leistungen, die von Institutionen garantiert werden, die medizinische Befunde erstellen und deren Identität in einem Erfassungsmodul 10d gespeichert sind. Die Leistungen umfassen im Falle des vorliegenden Ausführungsbeispiels eine Angabe über die Spezialisierung der Institution, eine Zeitdauer, die die Institution benötigt, um einen medizinischen Befund zu erstellen, eine Anzahl von medizinischen Befunden, die die Institution innerhalb einer vorgegebenen Zeitdauer erstellen kann, eine Gebühr, die die Institution für das Erstellen des medizinischen Befundes verlangt und für welche Länder die Institution gesetzlich einen medizinischen Befund erstellen darf.

Im Falle des vorliegenden Ausführungsbeispiels sind in der Fig. 1 ein Krankenhaus 20 und ein Ärzteteam 30 als eine Auswahl von Institutionen, die medizinische Befunde erstellen und deren Identität in dem Erfassungsmodul 10d gespeichert sind, dargestellt.

Die Datenbank 10 umfasst des Weiteren ein Weiterleitungsmodul 10e, in welchem der an die Datenbank 10 übermittelte Auftrag nötigenfalls an die ermittelte Institution weitergeleitet wird. Das Weiterleitungsmodul 10e ist darüber hinausgehend derart ausgeführt, dass die Identität des Auftraggebers, wenn gewünscht, der ermittelten Institution nicht preisgegeben wird. Auch eine Anonymisierung von personenbezogenen Daten derjenigen Personen, für die der medizinische Befund erstellt werden soll, ist in dem Weiterleitungsmodul 10e realisiert.

Die Speicher- und Weiterleitungsmodule 10a und 10e sind auch dafür vorgesehen, den von der Institution erstellten und an die Datenbank 10 übermittelten medizinischen Befund zu speichern und an den Auftraggeber weiterzuleiten. Die Identität der Institution kann auch geheim gehalten werden.

Mittels eines Abrechnungsmoduls 10f der Datenbank 10 kann eine Rechnung für den Auftraggeber aufgrund der im Leistungskatalog 10c gespeicherter Daten erstellt werden. Die Rechnung umfasst eine Gebühr für das Ermitteln der geeigneten Institution und nötigenfalls für das Weiterleiten des Auftrages und eine Gebühr für das Erstellen des medizinischen Befundes. Das Abrechnungsmodul 10f ist auch dafür vorgesehen, eine Bezahlung der ermittelten Institution für das Erstellen des medizinischen Befundes einzuleiten.

Damit der telemedizinische Dienstleister eine gleichbleibend hohe Qualität erstellter medizinischer Befunde sicherstellen kann, umfasst die Datenbank 10 ein Qualitätsmodul 10g, aufgrund dessen im Falle des vorliegenden Ausführungsbeispiels stichprobenartig Auftraggeber befragt werden, ob sie mit der Qualität der erstellten medizinischen Befunde zufrieden sind. Außerdem lässt das Qualitätsmodul 10g stichprobenartig medizinische Befunde aufgrund desselben medizinischen Datensatzes von verschiedenen Institutionen erstellen und vergleicht, ob die erstellten medizinischen Befunde vergleichbar sind, also zumindest zu ähnlichen Schlussfolgerungen kommen.

Sollte das in der Datenbank 10 gespeicherte Rechnerprogramm aufgrund eines Auftrages keine geeignete Institution ermitteln können, übermittelt sie dem Auftraggeber eine entsprechende Nachricht.

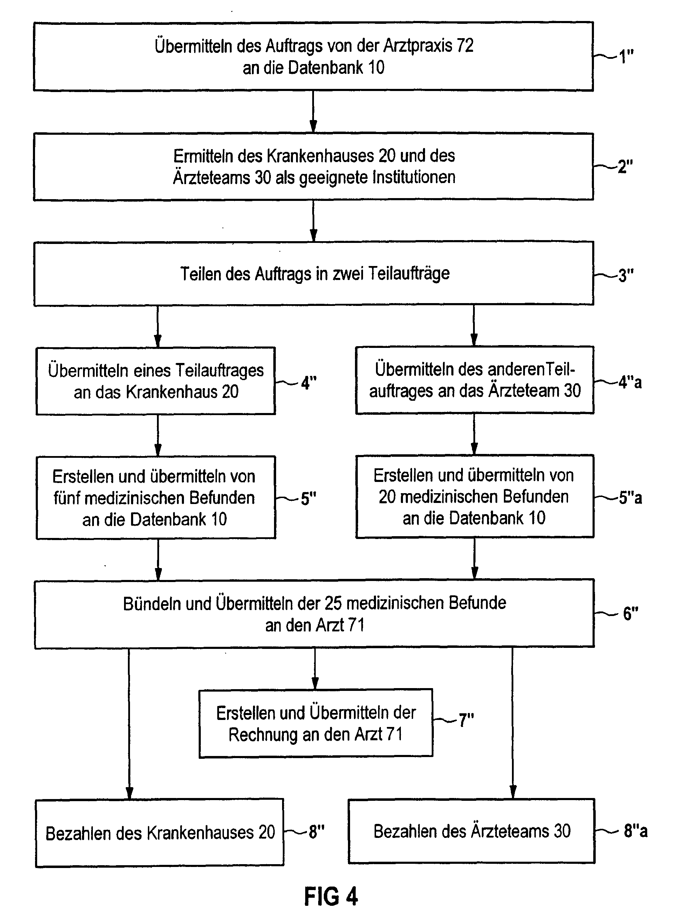

Die Fig. 1 zeigt außerdem einen weiteren Arzt 51, der ein mit einem in seiner Arztpraxis 52 befindlichen Röntgengerät 55 ein Röntgenbild 53 von einem Patienten 54 aufnahm. Das Röntgenbild 53 ist im Falle des vorliegenden Ausführungsbeispieles ein Röntgenbild 53 der Lunge des Patienten 54. Der Arzt 51 ist sich im Falle des vorliegenden Ausführungsbeispieles ebenfalls unschlüssig über eine Diagnose, so dass er mit einem in seiner Arztpraxis 52 befindlichen und an das Internet angeschlossenen Rechner 56 einen Auftrag an die Datenbank 10 übermittelt, er wolle einen medizinischen Befund erstellt bekommen. Der Auftrag umfasst im Falle des vorliegenden Ausführungsbeispieles einen dem Röntgenbild 53 zugeordneten Bilddatensatz (Schritt 1' des in der Fig. 4 dargestellten Flussdiagramms).

Ferner zeigt die Fig. 1 einen weiteren Arzt 61, der mit einem in seiner Arztpraxis 62 befindlichen Röntgengerät 65 ein Röntgenbild 63 von einem Patienten 64 aufnahm. Das Röntgenbild 63 ist im Falle des vorliegenden Ausführungsbeispieles ebenfalls ein Röntgenbild 63 der Lunge des Patienten 64. Der Arzt 61 ist sich ebenso unschlüssig über eine Diagnose, so dass er mit einem in seiner Arztpraxis 62 befindlichen und an das Internet angeschlossenen Rechner 66 einen Auftrag an die Datenbank 10 übermittelt, er wolle einen medizinischen Befund erstellt bekommen. Der Auftrag umfasst auch einen dem Röntgenbild 63 zugeordneten Bilddatensatz (Schritt 1'a des in der Fig. 4 dargestellten Flussdiagramms).

Im Falle des vorliegenden Ausführungsbeispieles erkennt das in der Datenbank 10 gespeicherte Rechnerprogramm, dass beide Ärzte 51 und 61 jeweils einen medizinischen Befund aufgrund eines Röntgenbildes 53 bzw. 63 einer Lunge erstellt bekommen wollen. Aufgrund der in dem Leistungskatalog 10c und dem Erfassungsmodul 10d gespeicherten Daten ermittelt das Vermittlungsmodul 10b des Rechnerprogramms, dass für das Krankenhaus 20 Lungenexperten 23 arbeiten. Das Krankenhaus 20 bietet außerdem eine Erstellung eines medizinischen Befundes aufgrund von Röntgenbildern einer Lunge dann besonders kostengünstig an, wenn der Auftrag eine Erstellung mehrerer medizinischer Befunde umfasst (Schritte 2' und 2'a des in der Fig. 4 dargestellten Flussdiagramms).

Daher fasst das in der Datenbank 10 gespeicherte Rechnerprogramm beide Aufträge der Ärzte 51 und 61 zu einem Sammelauftrag zusammen und übermittelt den Sammelauftrag an einen Rechner 22, der sich in dem Krankenhaus 20 befindet und an das Internet angeschlossen ist (Schritte 3' und 4' des in der Fig. 4 dargestellten Flussdiagramms). Der Sammelauftrag umfasst im Übrigen die den Röntgenbildern 53 und 63 zugeordneten Bilddatensätze, die in dem Speichermodul 10a aufgrund der von den Ärzten 51 und 61 übermittelten Aufträge gespeichert sind. Daraufhin erstellen die Lungenexperten 23 jeweils einen medizinischen Befund aufgrund des Sammelauftrages und übermitteln die erstellten medizinischen Befunde mit dem Rechner 22 an die Datenbank 10 (Schritte 5' und 6' des in der Fig. 4 dargestellten Flussdiagramms).

Das in der Datenbank 10 gespeicherte Rechnerprogramm übermittelt daraufhin den für den Arzt 51 bestimmten medizinischen Befund an den Rechner 56 des Arztes 51 (Schritt 7' des in der Fig. 4 dargestellten Flussdiagramms) und den für den Arzt 61 bestimmten medizinischen Befund an den Rechner 66 des Arztes 61 (Schritt 7'a des in der Fig. 4 dargestellten Flussdiagramms).

Aufgrund der in dem Abrechnungsmodul 10f gespeicherten Daten erstellt anschließend das in der Datenbank 10 gespeicherte Rechnerprogramm jeweils eine Rechnung für den Arzt 51 und 61 für die Vermittlung des Krankenhauses 20 als Institution, die für die Ärzte 51 und 61 einen medizinischen Befund erstellten. Die Rechnungen umfassen auch eine Gebühr für die Erstellung der medizinischen Befunde. Die jeweilige Rechnung wird danach an den Rechner 56 bzw. 66 übermittelt (Schritte 8' und 8'a des in der Fig. 4 dargestellten Flussdiagramms). Nachdem die Ärzte 51 und 61 jeweils ihre Rechnung beglichen haben, veranlasst das in der Datenbank 10 gespeicherte Rechnerprogramm aufgrund der in dem Abrechnungsmodul 10f gespeicherten Daten ein Bezahlung des Krankenhauses 20 für die erstellten medizinischen Befunde (Schritt 9' des in der Fig. 4 dargestellten Flussdiagramms).

Die Fig. 1 zeigt außerdem einen weiteren Arzt 71, der Mammographie-Reihenuntersuchungen (Screenings) durchführt. Zu diesem Zweck besitzt er ein geeignetes Röntgengerät 75, das sich in seiner Arztpraxis 72 befindet. Im Falle des vorliegenden Ausführungsbeispieles führt der Arzt 71 durchschnittlich 25 Mammographieuntersuchungen täglich durch, so dass er durchschnittlich 25 Mammogramme 73a bis 73y von 25 Patientinnen 74a bis 74y täglich mit dem Röntgengerät 75 erstellt. Aus Zeitgründen kann der Arzt 71 die Mammogramme 73a bis 73y nicht selber auswerten, so dass er im Falle des vorliegenden Ausführungsbeispieles mit einem in der Arztpraxis 72 befindlichen und an das Internet angeschlossenen Rechner 76 einen Auftrag an die Datenbank 10 übermittelt, 25 medizinische Befunde aufgrund der 25 Mammogramme 73a bis 73y zu erstellen. Der Auftrag des Arztes 71 umfasst auch den 25 Mammogrammen 73a bis 73y zugeordnete Bilddatensätze (Schritt 1" des in der Fig. 5 dargestellten Flussdiagramms).

Aufgrund des Auftrages des Arztes 71 ermittelt das in der Datenbank 10 gespeicherte Rechnerprogramm, dass ein Ärzteteam 30 auf ein Erstellen eines medizinischen Befundes aufgrund eines Mammogramms spezialisiert ist. Das Ärzteteam 30 kann jedoch nur 20 medizinische Befunde täglich erstellen. Ferner ermittelt das in der Datenbank 10 gespeicherte Rechnerprogramm, dass in dem Krankenhaus 20 weitere Ärzte 24 angestellt sind, die ebenfalls einen medizinischen Befund aufgrund eines Mammogramms erstellen können. Die Ärzte 24 des Krankenhauses 20 können täglich etwa zehn medizinische Befunde erstellen (Schritt 2" des in der Fig. 5 dargestellten Flussdiagramms).

Daraufhin teilt das in der Datenbank 10 gespeicherte Rechnerprogramm den Auftrag des Arztes 71 in zwei Teilaufträge und beauftragt das Krankenhaus 20, fünf medizinische Befunde zu erstellen und beauftragt das Ärzteteam 30, 20 medizinische Befunde zu erstellen (Schritt 3" des in der Fig. 5 dargestellten Flussdiagramms). Daher übermittelt die Datenbank 10 dem Rechner 22 des Krankenhauses 20 fünf Bilddatensätze, denen fünf Mammogramme zugeordnet sind (Schritt 4" des in der Fig. 5 dargestellten Flussdiagramms). Außerdem übermittelt die Datenbank 10 einem Rechner 32, welcher sich in einem Ärztehaus 31 des Ärzteteams 30 befindet und an das Internet angeschlossen ist, Bilddatensätze, denen 20 Mammogramme der Mammogramme 73a bis 73y zugeordnet sind (Schritt 4"a des in der Fig. 5 dargestellten Flussdiagramms).

Nachdem die Ärzte 24 ihre fünf medizinischen Befunde und das Ärzteteam 30 ihre 20 medizinischen Befunde erstellt haben, übermitteln beide ihre medizinischen Befunde an die Datenbank 10 (Schritte 5" und 5"a des in der Fig. 5 dargestellten Flussdiagramms). Die Datenbank 10 bündelt die 25 medizinischen Befunde und übermittelt sie anschließend wieder an den Rechner 76 des Arztes 71, so dass dieser die 25 medizinischen Befunde lesen kann (Schritt 6" des in der Fig. 5 dargestellten Flussdiagramms).

Aufgrund der in dem Abrechnungsmodul 10f gespeicherten Daten erstellt anschließend das in der Datenbank 10 gespeicherte Rechnerprogramm eine Rechnung für den Arzt 71 für die Vermittlung des Krankenhauses 20 und des Ärzteteams 30 als geeignete Institutionen. Die Rechnung umfasst auch eine Gebühr für die Erstellung der medizinischen Befunde. Die Rechnung wird danach an den Rechner 76 übermittelt (Schritt 7" des in der Fig. 5 dargestellten Flussdiagramms). Das in der Datenbank 10 gespeicherte Rechnerprogramm veranlasst aufgrund der in dem Abrechnungsmodul 10f gespeicherter Daten ein Bezahlung des Krankenhauses 20 und des Ärzteteams 30 (Schritte 8" und 8"a des in der Fig. 5 dargestellten Flussdiagramms).

Im beschriebenen Ausführungsbeispiel sind die Bilddatensätze, die den verschiedenen Röntgenbilder 53 und 63 sowie den Mamogrammen 73a bis 73y zugeordnet sind, medizinische Datensätze. Ein medizinischer Datensatz kann aber noch weitere Angaben umfassen. Er kann insbesondere einem Laborergebnis, einem Videofilm, einer Krankenakte eines Patienten zugeordnet sein. Der Bilddatensatz kann auch einem anderen Bild als einem Röntgenbild zugeordnet sein. Das Bild kann insbesondere mit einem Computertomograph, einem Magnetresonanzgerät oder mit einem Ultraschallgerät aufgenommen worden sein. Der medizinische Datensatz kann auch einem EKG zugeordnet sein.

Als Informationsübertragungsnetz zum Übermitteln des Auftrages, des medizinischen Befundes oder des medizinischen Datensatzes muss nicht unbedingt das Internet verwendet werden. Es kann insbesondere auch ein Telefonnetz Verwendung finden.

## Patentansprüche

1. Verfahren zum Ermitteln einer einen medizinischen Befund erstellenden Institution, aufweisend folgende Verfahrensschritte:
- Empfangen eines ersten Auftrages mit einer Datenbank (10), wobei der erste Auftrag über ein Informationsübertragungsnetz an die Datenbank (10) übermittelt wurde, der erste Auftrag einen ersten medizinischen Datensatz eines ersten Patienten (54, 64, 74a-74y) umfasst und der erste Auftrag ein Auftrag zum Erstellen eines ersten medizinischen Befundes aufgrund der ersten medizinischen Daten ist,
- basierend auf dem ersten Auftrag, automatisches Abfragen von in der Datenbank (10) gespeicherten Informationen über medizinische Befunde erstellende Institutionen (20, 30) aus einer Menge von Institutionen,
- basierend auf den abgefragten Informationen, Ermitteln einer geeigneten Institution (20, 30) aus der Menge der Institutionen, die den ersten medizinischen Befund aufgrund des ersten Datensatzes erstellen soll,
- Weiterleiten des ersten Auftrages mit dem ersten Datensatzes an die geeignete Institution.

2. Verfahren nach Anspruch 1, bei dem der erste Auftrag nach der Übermittlung an die Datenbank (10) in eine Liste eingetragen wird, wobei die Liste Aufträge umfasst, für die jeweils noch keine geeignete Institution ermittelt wurde, und wobei die Liste von Institutionen der Menge von Institutionen eingesehen werden kann, um ein Angebot zum Erstellen des medizinischen Befundes an die Datenbank (10) zu übermitteln.

3. Verfahren nach Anspruch 2, bei dem die Liste über das Internet aufgerufen werden kann.

4. Verfahren nach einem der Ansprüche 1 bis 3, bei dem der erste medizinische Befund von der den ersten medizinischen Befund erstellenden Institution (20, 30) an den Auftraggeber des ersten Auftrages übermittelt wird.

5. Verfahren nach Anspruch 4, bei dem die Datenbank (10) den ersten medizinischen Befund von der den ersten medizinischen Befund erstellenden Institution (20, 30) über das Informationsübertragungsnetz empfängt und an den Auftraggeber (51, 61, 71) des ersten Auftrags weiterleitet.

6. Verfahren nach einem der Ansprüche 1 bis 5, bei dem die Institution ein Arzt, ein Ärzteteam (30) und/oder ein Krankenhaus (20) ist.

7. Verfahren nach einem der Ansprüche 1 bis 6, bei dem der erste medizinische Datensatz einem Bild (53, 63, 73a-73y), einem Videofilm, Laborergebnisse des Patienten, einem EKG und/oder einer Krankenakte des ersten Patienten zugeordnet ist.

8. Verfahren nach Anspruch 7, bei dem das Bild mit einem bildgebenden medizintechnischen Gerät (55, 65, 75) hergestellt wurde.

9. Verfahren nach Anspruch 8, bei dem das bildgebende medizintechnische Gerät ein Computertomograph, ein Magnetresonanzgerät, ein Ultraschallgerät oder ein Röntgengerät (55, 65, 75) ist.

10. Verfahren nach einem der Ansprüche 1 bis 9, bei dem die geeignete Institution (20, 30) basierend auf wenigstens einem Kriterium ermittelt wird.

11. Verfahren nach Anspruch 10, bei dem das Kriterium eine geforderte Qualität des erstellten ersten medizinischen Befundes, eine medizinische Spezialisierung der Institution (20, 30), eine Zeitdauer zur Erstellung des ersten medizinischen Befundes, eine Anzahl von medizinischen Befunden, die die Institution (20, 30) in einer vorgegebenen Zeitdauer erstellen kann, von der Institution (20, 30) für die Erstellung des ersten medizinischen Befundes geforderte Gebühr und/oder gesetzliche Regelungen ist.

12. Verfahren nach Anspruch 10 oder 11, bei dem das Kriterium eine Qualität des an die Datenbank (10) übermittelten ersten medizinischen Datensatzes ist.

13. Verfahren nach einem der Ansprüche 1 bis 12, bei dem eine von der ermittelten Institution (20, 30) erbrachte Leistung von der Datenbank (10) überwacht wird.

14. Verfahren nach Anspruch 13, bei dem die erbrachte Leistung eine Qualität des ersten erstellten medizinischen Befundes, ein Einhalten einer vorgegebenen Zeitdauer für die Erstellung des ersten medizinischen Befundes und/oder eine Einhaltung einer Erstellung einer vorgegebenen Anzahl von medizinischen Befunden ist.

15. Verfahren nach einem der Ansprüche 1 bis 14, bei dem die Datenbank (10) automatisch eine Rechnung für das Erstellen des ersten medizinischen Befund generiert und an den Auftraggeber des ersten Auftrags übermittelt.

16. Verfahren nach einem der Ansprüche 1 bis 15, bei dem der erste Auftrag ein Auftrag zum Erstellen mehrerer medizinischer Befunde für mehrere Patienten (74a-74y) ist, der für die Erstellung der medizinischen Befunde notwendige medizinische Datensätze umfasst und die medizinischen Befunde von einer Institution oder von mehreren Institutionen (20, 30) aus der Menge von Institutionen (20, 30) erstellt werden.

17. Verfahren nach einem der Ansprüche 1 bis 16, bei dem zusätzlich die Datenbank (10) einen zweiten Auftrag zum Erstellen eines zweiten medizinischen Befundes eines zweiten Patienten (54, 64) über das Informationsübertragungsnetz empfängt, der zweite Auftrag einen für die Erstellung des zweiten medizinischen Befundes notwendigen zweiten medizinischen Datensatz des zweiten Patienten (54, 64) umfasst, der erste und der zweite Auftrag als ein Sammelauftrag zum Erstellen des ersten und des zweiten medizinischen Befundes von der Datenbank (10) an eine Institution (20) aus der Menge von Institutionen (20, 30) vergeben wird und der erste und der zweite medizinische Datensatz von der Datenbank (10) an die Institution (20) übermittelt wird.

18. Datenbank,
- die über ein Informationsübertragungsnetz kontaktierbar ist,
- an die ein Auftrag zum Erstellen eines medizinischen Befundes aufgrund eines den Auftrag umfassenden medizinischen Datensatzes eines Patienten (54, 64, 74a-74y) über das Informationsübertragungsnetz übermittelbar ist,
- in der Daten einer Menge von Institutionen (20, 30), die medizinische Befunde erstellen, gespeichert und abfragbar sind,
- die derart konfiguriert ist, dass sie automatisch die Daten der Menge von Institutionen (20, 30) mit dem Auftrag vergleicht und eine zum Erstellen des ersten medizinischen Befundes geeignete Institution (20, 30) aus der Menge von Institutionen (20, 30) auswählt, und
- und die derart konfiguriert ist, dass sie den medizinischen Datensatz über das Informationsübertragungsnetz an die ermittelte Institution (20, 30) weiterleitet.

19. Datenbank nach Anspruch 18, die Mittel (10f) zur Erstellung einer Rechnung aufweist, welche eine Rechnung für die Ermittlung der geeigneten Institution (20, 30) an den Auftraggeber (51, 61, 71) erstellen und übermitteln.

20. Datenbank nach Anspruch 18 oder 19, die Mittel (10g) zur Qualitätsüberprüfung des von der Institution (20, 30) erstellten medizinischen Befundes aufweist.
